# EUROPEAN PATENT SPECIFICATION

(11) **EP 3 181 168 B1**
(45) Date of publication and mention of the grant of the patent: **04.11.2020**
(21) Application number: 16204650.2
(22) Date of filing: 16.12.2016
(51) Int. Cl.: A61M 5/31, A61M 5/315

(54) **MANUALLY OPERATED INSTRUMENT FOR FILLING SYRINGES**
HANDBETÄTIGTE VORRICHTUNG ZUM BEFÜLLEN VON SPRITZEN.
INSTRUMENT A COMMANDE MANUELLE POUR LE REMPLISSAGE DES SERINGUES.

(30) Priority: 17.12.2015 IT UB20159178
(43) Date of publication of application: 21.06.2017
(73) Proprietor: CANE' S.P.A., 10098 Rivoli (TO) (IT)
(72) Inventor: CANE', Claudio, 10098 Rivoli (TO) (IT); CANE', Mario, 10098 Rivoli (TO) (IT); CANE', Paolo, 10098 Rivoli (TO) (IT)
(74) Representative: Robba, Pierpaolo

(56) References cited:
- EP-A1- 1 358 856
- US-A- 3 128 765
- US-A- 5 059 179

## Description

### Technical Field

The present invention relates to a manually operated instrument for filling syringes. More particularly, the invention relates to a method for filling syringes for medical use.

### Prior Art

It is known that, in medical field, use is made of syringes comprising a barrel, a tip for drug inlet and outlet located at one end of the barrel, a plunger slidable in the barrel and a shaft associated with the plunger in order to make the plunger slide in the barrel. The barrel usually has a cylindrical shape and is tapered in correspondence of the tip. The barrel end opposite to the tip is generally open to allow the plunger entering into the barrel. The syringes may have different internal barrel diameters, depending on the amount of liquid to be contained. For instance, syringes are known with a capacity of 50 and 100 ml and a length of about 50 mm. Such syringes have diameters of some centimetres and require a considerable effort in order to be loaded, because of the large volume of liquid being sucked into the syringe per unit of forward movement of the plunger within the barrel. Syringes for medical are generally made of transparent, medical-grade polypropylene plastics, preferably without any trace of PVC.

Generally, the plunger is made of an elastically deformable material, for instance rubber or the like, has a cylindrical shape and is tapered in the direction of the syringe tip. The external diameter of the plunger is chosen so that a sufficient tightness for enabling suction and injection of the liquid when the plunger is made to slide along the barrel is created between the plunger and the barrel. In order to make the plunger slide in the barrel, generally a control shaft removably associated with the plunger is provided. To this end, the plunger may be equipped with a rigid internal insert into which one of the ends of the control shaft engages. The control shaft must have a length greater than the barrel length: indeed, if the shaft projects from the barrel opening for entrance of the plunger, the user can seize and draw/push it in order to move the plunger and hence to suck/inject the liquid through the tip.

Moreover, the base of the syringe barrel can be equipped with radial blades in correspondence of the opening for entrance of the plunger. Such radial blades improve the grip for the user during suction and injection of the liquid. In some applications, such radial blades are exploited for coupling the syringe, without the shaft, with infusion pumps. Said pumps are actually provided with a female connector capable of receiving the radial blades of the syringe. EP 1078643 (A1) discloses an example of pumps of such kind, which can receive syringes with radial blades. Syringes having a bayonet connector, by means of which the syringe can be associated with an infusion pump, are also known. EP 2810673 (A1) discloses an example of syringes of such kind equipped with a bayonet connector and intended for infusion pumps.

Infusion pumps are electro-medical devices intender for the controlled administration of a liquid, usually a drug contained in the syringe.

Use of pumps for drug infusion is particularly advantageous for patients undergoing a prolonged therapy. Pharmacological therapies in which infusion pumps are used sometime require infusion of a drug for a very long time, or the infusion of a large amount of drug. In order to avoid frequent replacement of the syringe associated with the pump, syringes of large size have been developed, containing for instance 50 or 100 ml. Yet, with such volumes, the internal diameter of the syringe barrel is large, with the consequence that manual filling of the syringes with the drug to be injected is very tiring and sometime impossible for a weakened or aged person. Actually, when the internal barrel diameter is so large, the resistance opposed by the plunger to its sliding within the barrel is such that the pulling force to be applied to make the plunger slide is high. The two parameters are indeed proportional: the larger the diameter, the higher the pulling force to be applied. It is therefore obvious that, if such kind of application is difficult for wholly healthy persons, it will be even more difficult, or even impossible, for patients who are by their nature weakened.

Moreover, often the drugs to be injected are contained in so-called vials, consisting of small vessels of glass or plastics closed by a pierceable membrane, e.g. of Teflon®. The presence of such a membrane prevents air from entering the vial while the drug is being sucked. This results in a difficulty in drug suction into the syringe, since a vacuum condition is generated that opposes to drug suction into the syringe.

A further difficulty factor arises when the drug to be sucked is characterised by a high viscosity. The more the drug is viscous, the higher is the pulling force the patient is to apply to the plunger to perform drug suction.

The above problems related to the barrel diameter, the sucking from a vial and the drug viscosity can be independent from one another, but they may even combine together: actually a drug can be viscous, can be contained in a vial and can demand a high dosage (e.g. 50 or 100 ml). In such circumstances, it is therefore readily apparent that the difficulty the patient encounters when sucking the drug with the syringe will be very high.

In order to overcome these problems, different devices have been disclosed aiming a making filling of a syringe easier. Two documents are mentioned here by way of examples, namely WO 02070049 and GB 2351907. WO 02070049 discloses some embodiments of instruments for filling syringes, having the common feature of providing a seat in the plunger for the engagement of a removable shaft. The instruments disclosed in the document exploit a lever assembly to impart the force necessary for suction. Such solutions are cumbersome, difficult to be handled and scarcely practical if the patient needs to bear with him/her the instrument for filling again the syringe, e.g. during a journey. GB 2351907 discloses a motor-operated instrument for filling syringes. That motor causes the rotation of a threaded screw connected to a support secured to the free end of the syringe shaft. The rotation of the screw is converted into a rectilinear motion of the shaft, resulting in a sliding of the plunger and the filling of the syringe. This motor-operated instrument is therefore cumbersome and scarcely practical when it is to be transported, and moreover its fabrication is expensive. EP 1 358 856, discloses a syringe provided with a sliding piston and rotative means which can be manually operated for shifting said piston and causing a linear non rotative shifting of the piston within the syringe barrel.

It is a main object of the present invention to provide a method for filling syringes using a manually operated instrument, which overcomes those drawbacks of the prior art. It is another object of the invention to provide a method that makes filling of syringes easier and reduces the effort required to fill a syringe with large diameter, thereby enabling also a weakened person to carry out such operation

It is a further, but not the last object of the invention to provide an instrument of the above kind, which can be industrially manufactured with limited costs.

The above and other objects are achieved by means of a manually operated instrument for filling syringes as claimed in the appended claims.

### Description of the invention

The method according to the invention enables, through a manual operation, easily filling syringes, even of great size with large diameters. The advantageous use of a manually-operated instrument avoids the high manufacturing costs that would be demanded by a motor-operated device. The instrument allows filling syringes having a plunger provided with a seat for a shaft controlling sliding of said plunger within the barrel of the syringe. Said instrument is equipped with means for coupling to said seat of the plunger. Once the coupling between the instrument and the plunger has occurred, application to the shaft of a pull force in a direction away from the syringe tip by means of a turnable grip nut will result in the plunger moving back away from the tip, thereby enabling the liquid to enter into the syringe through the tip. Preferably, the coupling between the instrument and the plunger is not permanent: indeed, at any time, it will be possible to decouple the instrument from the plunger. The instrument according to the invention allows filling a syringe with a given amount of liquid depending on the stop position of the plunger stroke. According to a preferred embodiment of the invention, the plunger stroke during syringe filling is limited by the instrument itself. In this manner, the syringe plunger is advantageously prevented from coming out through the opening provided in the barrel for entrance of the plunger. Moreover, at the end of the syringe filling, it will be advantageously possible to remove the instrument and, if necessary, to connect the syringe to an infusion pump or to a manual control shaft, in order to inject the liquid contained in the syringe.

In the instrument according to the invention the means for coupling to the seat of the plunger preferably include a threaded head or a head provided with ratchet means or a knurled head, firmly attached to one of the ends of a shaft, which is advantageously externally threaded. Moreover, the instrument according to the invention advantageously includes an actuating grip nut provided with an internally threaded through-hole through and with which said threaded shaft passes and comes into engagement.

The advantage of firmly attaching said head to one end of an externally threaded shaft and of providing an actuating grip nut provided with an internally threaded through-hole through and with which said threaded shaft passes and comes into engagement, consists in the capability to convert the rotary motion of the grip nut to a linear motion of the threaded shaft and hence of the plunger associated therewith. In order to perform such a conversion, the pitch of the thread of the threaded shaft must be the same as the pitch of the internal thread provided in the through-hole of the actuating grip nut. Having the same pitch causes the rotation of the grip nut to be converted to a linear motion of the shaft and the plunger when the relative axial motion of the barrel and the grip nut is prevented. In a first embodiment of the invention, said relative motion is prevented in that the grip nut abuts against the edge of the base of the syringe barrel at the opening for entrance of the plunger into the barrel. In a second embodiment of the invention, said relative motion is prevented in that the grip nut abuts against a ferrule located between the grip nut and the syringe barrel.

In this way, through a simple rotation of the grip nut, the filling of syringes even having a large internal diameter of the barrel is performed. The pitch of the thread and the diameter of the grip nut determine the rotation torque that is to be applied to make the plunger slide. The finer the pitch, the longer the time required for filling the syringe, but the smaller the torque to be applied to the grip nut.

The pitch of the screw can be chosen on a case by case basis depending of the diameter of the syringe barrel and the viscosity of the drug to be sucked.

For instance, for a syringe with a capacity of 100 ml and plunger stroke of about 50 mm, the pitch of the screw is preferably chosen out of 2 and 3 mm, preferably 2 mm, so that the grip nut is to make about 15 to 40 revolutions in order to fill the syringe.

According to the invention, said grip nut preferably has a circular shape and is provided with radially extending protrusions aimed at facilitating grip and turning of the grip nut by the user. Said grip nut preferably further comprises an abutment surface for the opening for entrance of the plunger into the syringe barrel. Preferably, said abutment surface has a frusto-conical shape. Such a shape enables the grip nut to abut against the opening for entrance of the plunger into the barrel, thus blocking the displacement of the plunger, at the end of the syringe filling operation, before the plunger can come out from the syringe barrel. Moreover, the frusto-conical shape, with such a size that the small base of the surface slightly penetrates into the syringe barrel through the opening for entrance of the plunger, prevents the grip nut from laterally displacing when it is turned by the user in order to fill the syringe.

Moreover, the instrument according to the invention preferably comprises a knob provided on the threaded shaft, at its end opposite to the coupling means. The advantageous function of said knob is to make grip by the user of the threaded shaft easier, in particular to make the engagement of the coupling means with the plunger seat easier.

In a second embodiment of the invention, the instrument comprises a supporting annular ferrule having a through-hole for the sliding of the threaded shaft. Said through-hole enables the shaft to freely pass through the ferrule, substantially without contact. Preferably, said annular ferrule has an abutment surface for the abutment surface of the grip nut and an engagement surface for the syringe. Preferably, said engagement surface for the syringe comprises a bayonet-like fitting. In this embodiment of the invention it is the ferrule, through the engagement surface for the syringe, which blocks the displacement of the plunger, at the end of the syringe filling operation, before the plunger can come out from the syringe barrel.

The instrument according to the invention is preferably made of plastic material.

The invention advantageously provides a manually operated instrument for filling syringes where the reduced number of components advantageously makes it an instrument of reduced and pocket size, easy to handle and easy and cheap to manufacture.

### Brief Description of the Figures

Some preferred embodiments of the invention will be described hereinafter by way of non-limiting example, with reference to the accompanying figures, in which:
- Fig. 1 is a perspective view of a first embodiment of the instrument according to the invention;
- Fig. 2A is a side view of the instrument shown in Fig. 1, in operating configuration, when the grip nut abuts against the base of the barrel of the syringe having an opening for entrance of the plunger;
- Fig. 2B is a view similar to Fig. 2A, in longitudinal section taken along line AA in Fig. 2A;
- Fig. 3A is a side view of a second embodiment of the instrument according to the invention, in operating configuration, when the grip nut abuts against the abutment surface of the supporting ferrule;
- Fig. 3B is a view similar to Fig. 3A, in longitudinal section taken along line AA in Fig. 3A.

### Description of some Preferred Embodiments

In all Figures, the same reference numerals have been used to denote equal or functionally equivalent components.

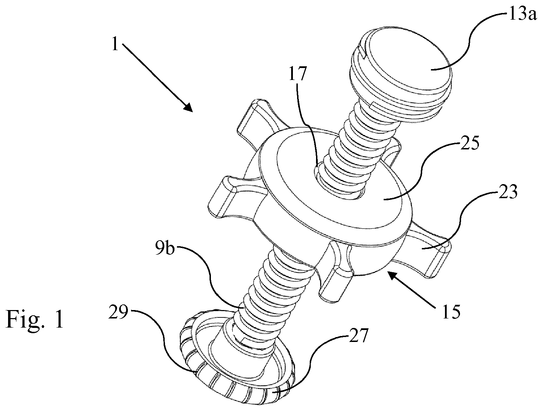

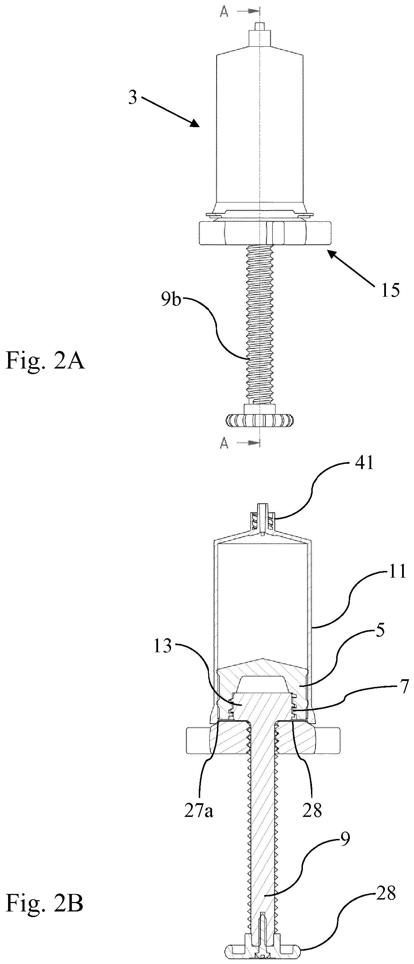

Referring to Figs. 1, 2A and 2B, a first embodiment of instrument 1 according to the invention is described. Said instrument 1 enables filling syringes 3 through a manual operation. Said syringes 3 include a plunger 5 provided with a seat 7 for the engagement of a shaft 9 controlling sliding of said plunger 5 within barrel 11 of syringe 3. Preferably, said seat 7 includes a cylindrical axial cavity with circular cross section. Said instrument 1 includes coupling means 13 capable of engaging with seat 7 of plunger 5. Instrument 1 according to the invention further includes coupling means 13 preferably including a threaded head 13a, firmly attached to one of the ends of shaft 9, which preferably is provided with a thread 9b. Preferably, said head 13a has a cylindrical shape with circular cross section and size complementary to, preferably slightly greater than, the size of seat 7 of plunger 5. In well-known manner, plunger 5 is made of an elastically deformable material, generally rubber or the like. On the one side, this enables plunger 5 to create the necessary tightness with the internal walls of barrel 22, and on the other side enables keeping head 13a in seat 7. Moreover, plunger 5 can be equipped with a threaded internal rigid insert into which head 13a engages. Moreover, instrument 1 according to the invention advantageously includes an actuating grip nut 15 provided with an internally threaded through-hole 17 through and with which said threaded shaft 9 passes and comes into engagement. Pitch 19 of threaded shaft 9 must be the same as pitch 21 of the internal thread provided in through-hole 17 of actuating grip nut 15. When grip nut 15 comes into abutment against edge 27a of opening 27 at the base of syringe barrel 11 and head 13a is engaged with seat 7 in the plunger, rotation of grip nut 15 makes threaded shaft 9 axially slide inside hole 17 of grip nut 15. Such operation enables an advantageous and easy filling also of syringes with large internal diameter of barrel 11, such as for instance syringes with a capacity of 50 or 100 ml and a plunger stroke of about 50 mm, which have therefore an internal diameter of some centimetres. Those syringes are capable of sucking about 1 to 2 ml liquid per mm of plunger stroke, and they would therefore require a considerable effort for being filled by applying a pulling force onto the plunger. During filling of syringe 3, the rotary motion of grip nut 15 is not transmitted to shaft 9 and hence to the plunger, thanks to the friction generated between the inner walls of barrel 11 and the external side surfaces of plunger 5. That frictional force is indeed greater than the rotation force imposed by grip nut 15. Consequently, during rotation of grip nut 15, neither shaft 9 nor plunger 5 will rotate relative to barrel 11, but they will only slide within it.

According to the invention, said grip nut 15 has a circular shape and is provided with protrusions or pegs 23 radially extending around grip nut 15. Pegs 23 aim at facilitating gripping and turning of grip nut 15 by the user. Said pegs 23 preferably have a longitudinally fitting shape, that is a shape becoming thinner along the side edges according to a curvilinear profile. Said grip nut 15 further comprises an abutment surface 25 for edge 27a of opening 27 for entrance of plunger 5 into barrel 11 of syringe 3. Preferably, said abutment surface 25 has a frusto-conical shape and enables blocking the back stroke of plunger 5, at the end of the filling of syringe 3, thereby preventing the plunger from going out from barrel 11 of syringe 3.

Instrument 1 according to the invention preferably further includes a knob 28 provided on threaded shaft 9 at end 13b opposite to head 13a. Preferably, said knob 28 is disc shaped and has radial protrusions 29 aimed at facilitating grip of knob 28 by the user. The function of knob 28 is to make coupling head 13a into seat 7 of plunger 5 easier.

The operating configuration shown in Figs. 2A and 2B is attained at the end of the filling of syringe 3. In order to attain such a configuration, first of all head 13a is to be coupled into seat 7 of plunger 5 (which in such phase will be located in correspondence of tip 41 of syringe 3) with the aid of knob 28, and grip nut 15 is to be turned, with the aid of pegs 23, until plunger 5 stops when abutting against abutting surface 25. Through such a simple operation, it is advantageously possible to fill large size syringes, such as syringes with capacity of 50 or 100 ml and diameter of some centimetres, with little effort. At the end of the syringe filling, instrument 1 can be detached form syringe 3 by decoupling head 13a from seat 7 of plunger 5, thus enabling advantageously the syringe filled with liquid to be connected to an infusion pump for injecting the liquid, over time, to the patient.

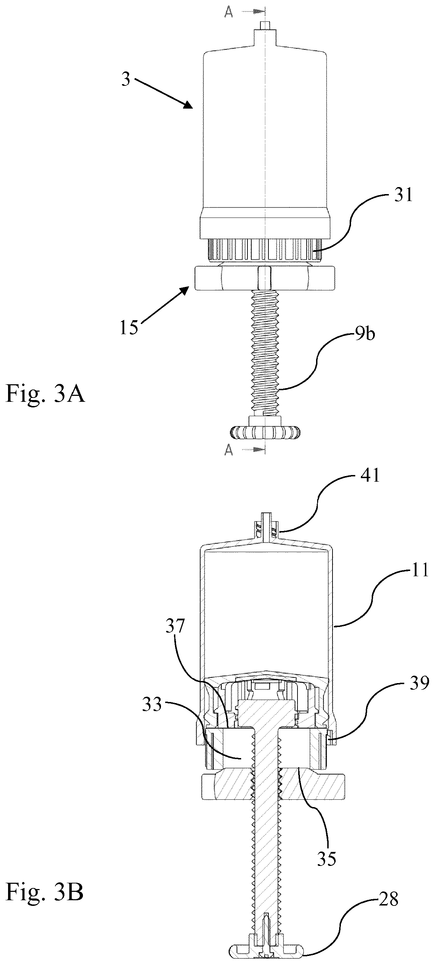

A second embodiment of the invention is described with reference to Figs. 3A and 3B.

In this second embodiment of the invention, instrument 1 includes a supporting annular ferrule 31 having a through-hole 33 for the sliding of the threaded shaft 9. Said hole 33 enables threaded shaft 9 to freely pass through ferrule 31 without contact. Preferably, said annular ferrule 31 has an abutment surface 35 for abutment surface 25 of grip nut 15 and an engagement surface 37 for syringe 3. Said engagement surface 37 can comprise a bayonet-like connector 39. In this embodiment of the invention it is ferrule 31, through engagement surface 27 for syringe 3, which blocks the displacement of plunger 5, at the end of the syringe filling operation, before the plunger can come out from barrel 11. Moreover, ferrule 31 laterally retains grip nut 15 thanks to the fact that frusto-conical surface 25 slightly penetrates into hole 33 of ferrule 31 and abuts against the internal wall of said hole when ferrule 31 is being turned by the user in order to cause drug suction into the syringe.

The instrument as described and shown can undergo several changes and modifications, included in the same inventive principle.

## Claims

1. Method for filling syringes (3) having a plunger (5) made of an elastically deformable material provided with a seat (7) for a removable control shaft controlling sliding of said plunger within the barrel (11) of the syringe, said barrel comprising an opening (27) for entrance of plunger (5) provided with an edge (27a), said method comprising the steps of:
- providing an instrument removably coupled to the syringe and equipped with:
- coupling means (13a) for removably coupling said instrument to said seat (7) of the plunger (5), said coupling means being firmly attached to one of the ends of an externally threaded shaft (9),
- an actuating grip nut (15) provided with an internally threaded through-hole (17) through which said threaded shaft (9) passes and comes into engagement, said internally threaded through-hole (17) provided in the actuating grip nut (15) comprising a thread having the same pitch as the thread (9b) of the threaded shaft (9);
- an optional supporting annular ferrule (31) having a through-hole (33) for the sliding of the threaded shaft (9), said supporting annular ferrule (31) comprising an engagement surface (37) for the barrel (11) of the syringe (3);
- engaging said coupling means with said seat (7) of the elastically deformable plunger (5);
- causing said grip nut (15) to come into abutment against edge (27a) of opening (27) of the base of the syringe barrel (11) at the opening for entrance of the plunger (5) into the barrel or, if present, against said optional ferrule (31) located between the grip nut (15) and the syringe barrel (11);
- rotating said grip nut (15), when the coupling means (13a) are engaged within the seat (7) of the plunger (5) and the actuating grip nut (15) is in contact with the barrel (11) of the syringe or with said ferrule, thus causing threaded shaft (9) to axially slide inside hole (17) of grip nut (15) and withdrawal of the plunger (5) in the barrel (11) of the syringe, this resulting in suction of liquid through the syringe tip.

2. Method according to claim 1, wherein said coupling means (13a) for coupling to said seat (7) of the plunger (5) comprise a threaded head or a head provided with ratchet means or with knurling.

3. Method according to claim 2, wherein said grip nut (15) has a circular shape and comprises protrusions (23) extending radially around the grip nut (15).

4. Method according to claim 3, wherein said grip nut (15) comprises a frusto-conical abutment surface (25) for the base of the syringe barrel, said surface being provided with an opening for entrance of the plunger.

5. Method according to claim 4, wherein said threaded shaft (9) comprises a knob (28) at its end opposite to the coupling means (13a) for coupling to said seat (7) of the plunger (5).

6. Method according to claim 1, wherein said engagement surface (37) of the annular ferrule (31) for the syringe comprises a bayonet-like insert (39).

## Patentansprüche

1. Verfahren zum Befüllen von Spritzen (3), die einen Kolben (5) aufweisen, der aus einem elastisch deformierbarem Material hergestellt ist und mit einem Sitz (7) für einen abnehmbaren Steuerschaft, der das Gleiten des Kolbens in dem Zylinder (11) der Spritze steuert, versehen ist, wobei der Zylinder eine Öffnung (27) für das Eindringen des Kolbens (5) aufweist, die einen Rand (27a) aufweist, wobei das Verfahren die folgenden Schritte aufweist:
- Bereitstellen eines Instruments, das lösbar an die Spritze gekoppelt und ausgestattet ist mit:
- Kopplungsmitteln (13a) zum abnehmbaren Ankoppeln an den Sitz (7) des Kolbens (5), wobei die Kopplungsmittel fest an einem der Enden eines Schafts (9), der ein Außengewinde aufweist, angebracht sind,
- einer griffigen Betätigungsmutter (15), die mit einem Durchgangsloch (17) versehen ist, das ein Innengewinde aufweist, durch welches der mit einem Gewinde versehene Schacht (9) hindurchragt und in das er eingreift, wobei das mit einem Innengewinde versehene Durchgangsloch (17), das in der griffigen Betätigungsmutter (15) vorgesehen ist, ein Gewinde aufweist, das dieselbe Steigung wie das Gewinde (9b) des Schafts (9) hat,
- einem optionalen stützenden kreisförmigen Endring (31), der eine Durchgangsöffnung (33) zum Durchlass des mit einem Gewinde versehenen Schafts (9) aufweist, wobei der stützende kreisförmige Endring (31) eine Anlagefläche (37) für den Zylinder (11) der Spritze (3) aufweist,
- Koppeln der Kopplungsmittel mit dem Sitz (7) des elastisch verformbaren Kolbens (5),
- Bewirken, dass die griffige Mutter (15) an dem Rand (27a) der Öffnung (27) der Basis des Spritzenzylinders (11) an der Öffnung zum Eintreten des Kolbens (5) in den Zylinder, oder, falls vorhanden, an dem optionalen Endring (31), der zwischen der griffigen Mutter (15) und dem Zylinder (11) der Spritze angeordnet ist, zur Anlage kommt,
- Drehen der griffigen Mutter (15), wenn die Kopplungsmittel (13a) an den Sitz (7) des Kolbens (5) gekoppelt sind und die griffige Betätigungsmutter (15) mit dem Zylinder (11) der Spritze oder mit dem Endring Kontakt hat, was bewirkt, dass der mit einem Gewinde versehene Schaft (9) axial in dem Loch (17) der griffigen Mutter (15) gleitet und der Kolben (5) aus dem Zylinder (11) der Spritze gezogen wird, was zu einem Ansaugen von Flüssigkeit durch die Spitze der Spritze führt.

2. Verfahren nach Anspruch 1, wobei die Kopplungsmittel (13a) zum Ankoppeln an den Sitz (7) des Kolbens (5) einen Kopf mit einem Gewinde oder einen Kopf mit Ratschenmitteln oder mit Rändelung aufweisen.

3. Verfahren nach Anspruch 2, wobei die griffige Mutter (15) kreisförmig ist und Vorsprünge (23) aufweist, die radial von der griffigen Mutter (15) ausgehen.

4. Verfahren nach Anspruch 3, wobei die griffige Mutter (15) eine kegelstumpfförmige Anlagefläche (25) für die Basis des Spritzenzylinders aufweist, wobei die Fläche mit einer Öffnung für den Eintritt des Kolbens versehen ist.

5. Verfahren nach Anspruch 4, wobei der mit einem Gewinde versehene Schaft (9) an seinem Ende, das den Kopplungsmitteln (13a) zum Ankoppeln an den Sitz (7) des Kolbens (5) gegenüber liegt, einen Knauf (28) aufweist.

6. Verfahren nach Anspruch 1, wobei die Kopplungsfläche (37) des kreisförmigen Endrings (31) für die Spritze einen bajonettartigen Einsatz (39) aufweist.

## Revendications

1. Procédé de remplissage de seringues (3) ayant un piston (5) réalisé en un matériau déformable élastiquement pourvu d'un siège (7) pour la commande par une tige de commande amovible d'un coulissement dudit piston au sein du cylindre (11) de la seringue, ledit cylindre comprenant une ouverture (27) d'entrée de piston (5) pourvue d'un bord (27a), ledit procédé comprenant les étapes de :
- fourniture d'un instrument couplé amovible à la seringue et équipé :
- de moyens de couplage (13a) pour coupler de façon amovible ledit instrument audit siège (7) du piston (5), lesdits moyens de couplage étant attachés fermement à l'une des extrémités d'une tige filetée de manière externe (9),
- d'un écrou de serrage d'actionnement (15) pourvu d'un trou traversant fileté de manière interne (17) à travers lequel ladite tige filetée (9) passe et vient en engagement, ledit trou traversant fileté de manière interne (17) prévu dans l'écrou de serrage d'actionnement (15) comprenant un filet ayant le même pas que le filet (9b) de la tige filetée (9) ;
- une virole annulaire de support facultative (31) ayant un trou traversant (33) pour le coulissement de la tige filetée (9), ladite virole annulaire de support (31) comprenant une surface d'engagement (37) pour le cylindre (11) de la seringue (3) ;
- engagement desdits moyens de couplage avec ledit siège (7) du piston déformable élastiquement (5) ;
- mise en butée dudit écrou de serrage (15) contre le bord (27a) de l'ouverture (27) de la base du cylindre de seringue (11) au niveau de l'ouverture d'entrée du piston (5) dans le cylindre ou, le cas échéant, contre ladite virole facultative (31) située entre l'écrou de serrage (15) et le cylindre de seringue (11) ;
- rotation dudit écrou de serrage (15), lorsque les moyens de couplage (13a) sont engagés au sein du siège (7) du piston (5) et l'écrou de serrage d'actionnement (15) est en contact avec le cylindre (11) de la seringue ou avec ladite virole, provoquant ainsi le coulissement axial de la tige filetée (9) à l'intérieur du trou (17) de l'écrou de serrage (15) et le retrait du piston (5) dans le cylindre (11) de la seringue, avec pour résultat une aspiration de liquide à travers l'embout de seringue.

2. Procédé selon la revendication 1, dans lequel lesdits moyens de couplage (13a) pour un couplage audit siège (7) du piston (5) comprennent une tête filetée ou une tête pourvue d'un moyen de cliquet ou d'un moletage.

3. Procédé selon la revendication 2, dans lequel ledit écrou de serrage (15) a une forme circulaire et comprend des protubérances (23) s'étendant radialement autour de l'écrou de serrage (15).

4. Procédé selon la revendication 3, dans lequel ledit écrou de serrage (15) comprend une surface de butée tronconique (25) pour la base du cylindre de seringue, ladite surface étant pourvue d'une ouverture d'entrée du piston.

5. Procédé selon la revendication 4, dans lequel ladite tige filetée (9) comprend un bouton (28) au niveau de son extrémité opposée aux moyens de couplage (13a) pour un couplage audit siège (7) du piston (5).

6. Procédé selon la revendication 1, dans lequel ladite surface d'engagement (37) de la virole annulaire (31) pour la seringue comprend un insert de type baïonnette (39).
